# EUROPEAN PATENT APPLICATION

(11) **EP 4 844 002 A1**
(43) Date of publication of application: **25.11.2026**
(21) Application number: 25742018.2
(22) Date of filing: 17.01.2025
(51) Int. Cl.: C07K 16/18, C07K 1/22, C07K 16/22, C07K 16/24, C07K 16/26, C07K 16/28, C07K 16/40, C12N 15/13, C12P 21/08, C12Q 1/06, C12Q 1/68, C40B 40/08, C40B 40/10, G01N 33/53, G01N 33/573, G01N 33/574, G01N 33/577

(54) **METHOD FOR IDENTIFYING COMMON L CHAIN FOR USE IN PRODUCTION OF MULTISPECIFIC ANTIBODY**

(30) Priority: 19.01.2024 JP 2024006564
(71) Applicant: Perseus Proteomics Inc., Tokyo 103-0015 (JP)
(72) Inventor: KONDO Mariko, Tokyo 103-0015 (JP); OYA Junpei, Tokyo 103-0015 (JP); UKAI Yoshinori, Tokyo 103-0015 (JP)
(74) Representative: Hoffmann Eitle
(86) International application number: PCT/JP2025/001321
(87) International publication number: WO 2025/154793

(57) **Abstract**

It is an object of the present invention to provide a method for identifying a common L chain for use in production of a multispecific antibody. The present invention provides a method for identifying a common L chain for use in production of a multispecific antibody, comprising: (1) a step of producing a first library containing multiple antibodies that recognize a first antigen, (2) a step of producing a second library containing multiple antibodies that recognize a second antigen, and (3) a step of selecting an antibody that recognizes the first antigen and an antibody that recognizes the second antigen, which have a common L chain, from the first library and the second library.

## Description

### Technical Field

The present invention relates to a method for identifying a common L chain for use in production of a multispecific antibody. The present invention further relates to a method for producing a multispecific antibody.

### Background Art

An antibody consists of two different genes of an H chain and an L chain, and forms an antigen-binding molecule by a pair of constituent units. On the other hand, a multispecific antibody is an artificial protein that simultaneously binds to two or more types of different antigens. In recent years, there has been an increasing trend in the development of multispecific antibodies as a technique related to antibodies.

A challenge in the development of multispecific antibodies may be the need for different H chain and L chain pairs for each specific antibody, resulting in a complex expression structure. In the case of a bispecific antibody, two types of H chains and two types of L chains are required, and in order to obtain a correct combination, complicated steps are quired during expression and purification. Therefore, efforts are being made to commonize the L chains of antibodies.

For example, Non-Patent Document 1 discloses that a common L chain is found by modifying the L chains for two antigens to which a bispecific antibody binds. Specifically, a chimeric L chain was created genetically by shuffling the amino acid sequences of individual L chains, and then, by allowing it to co-express with each H chain and selecting the L chain that maintains binding to the antigen of interest, a common L chain was successfully obtained.

Moreover, it is known that a light chain library is used to obtain a common light chain (Non-Patent Document 2 and Non-Patent Document 3). In Non-Patent Document 3, the natural antibody diversification process that occurs in B cells is mimicked, and antibodies with high binding activity are obtained by a step of introducing mutations into light chains that are directly isolated from the library and achieving affinity maturation. Also, in Non-Patent Document 2, the repertoire size of the L chain library is given as 1.1 x 10⁵ and 1.7 x 10⁶, while in Non-Patent Document 3, there is no specific information such as the library size for the L chain library. Furthermore, since both methods use heavy chains derived from the library to achieve light chain commonality, the methods are disadvantageous in that they are poor in terms of versatility for utilizing a common light chain.

### Prior Art Documents

### Non-Patent Documents

Non-Patent Document 1: Zenjiro Sampei et al., PLos One 8 e57479 (2013)
Non-Patent Document 2: Nat Biotechnol. 1998 Jul; 16(7): 677-81.
Non-Patent Document 3: MABS. 2018, VOL. 10, NO. 2, 256-268

### Summary of Invention

### Objects to be Solved by the Invention

While efforts have been made to commonize the L chain of antibodies in production of multispecific antibodies, a highly versatile technique has yet to be established. It is an object of the present invention to provide a method for identifying a common L chain for use in production of a multispecific antibody. Furthermore, it is another object of the present invention to provide a method for producing a multispecific antibody, using a common L chain identified by the above-described method.

### Means for Solving the Objects

As a result of intensive studies directed towards achieving the above-described objects, the present inventors have found that a first library containing multiple antibodies that recognize a first antigen, and a second library containing multiple antibodies that recognize a second antigen, are each produced, and then, from the first library and the second library, an antibody that recognizes the first antigen and an antibody that recognizes the second antigen, which have a common L chain, are selected, so that the common L chain for use in production of a multispecific antibody can be identified. One of the features of the present invention is that even in a case where heavy chains derived from mouse immunity or heavy chains derived from human antibodies produced by humanized mice are used, novel combinations can be selected by screening an identical light chain library, making it extremely versatile. In the present invention, the binding activity actually measured using ELISA after screening shows a signal strength equal to or higher than that of the original sequence. In other words, the method of the present invention is very efficient in that the isolated light chain can be utilized as is. The present invention has been completed based on the above-described findings. According to the present invention, the following inventions are provided.

< 1 > A method for identifying a common L chain for use in production of a multispecific antibody, comprising:
   (1) a step of producing a first library containing multiple antibodies that recognize a first antigen,
   (2) a step of producing a second library containing multiple antibodies that recognize a second antigen, and
   (3) a step of selecting an antibody that recognizes the first antigen and an antibody that recognizes the second antigen, which have a common L chain, from the first library and the second library.
< 2 > The method according to < 1 > above, wherein
   the step (1) is a step of selecting multiple antibodies that recognize a first antigen from a library containing multiple antibodies having H chains and L chains, so as to produce a first library, and
   the step (2) is a step of selecting multiple antibodies that recognize a second antigen from a library containing multiple antibodies having H chains and L chains, so as to produce a second library.
< 3 > The method according to < 2 > above, wherein the library containing multiple antibodies having H chains and L chains contains 10⁹ types or more antibodies.
< 4 > The method according to < 2 > above, wherein the type of H chains in the library containing multiple antibodies having H chains and L chains is 10⁶ or more.
< 5 > The method according to < 2 > above, wherein the type of L chains in the library containing multiple antibodies having H chains and L chains is 10³ or less.
< 6 > The method according to < 2 > above, wherein the ratio between the types of H chains and the types of L chains in the library containing multiple antibodies having H chains and L chains is 1000 times or more.
< 7 > The method according to < 1 > above, wherein
   the step (1) comprises:
      (1-A) a step of incorporating nucleic acids encoding the H chains of the antibodies that recognize a first antigen into a library of nucleic acids encoding the L chains, to produce an antibody library containing the H chains and L chains of the antibodies that recognize the first antigen, and
      (1-B) a step of selecting the antibodies that recognize the first antigen from the antibody library obtained in the step (1-A), and
   the step (2) comprises:
      (2-A) a step of incorporating nucleic acids encoding the H chains of the antibodies that recognize a second antigen into a library of nucleic acids encoding the L chains, to produce an antibody library containing the H chains and L chains of the antibodies that recognize the second antigen, and
      (2-B) a step of selecting the antibodies that recognize the second antigen from the antibody library obtained in the step (2-A).
< 8 > The method according to < 7 > above, wherein the types of L chains in the antibody library obtained in the step (1-A) and in the antibody library obtained in the step (2-A) are each 10³ or less.
< 9 > The method according to < 1 > above, wherein
   the step (1) is a step of selecting multiple antibodies that recognize a first antigen from a library containing multiple antibodies having H chains and L chains, so as to produce a first library, and
   the step (2) comprises:
      (2-A) a step of incorporating nucleic acids encoding the H chains of the antibodies that recognize a second antigen into a library of nucleic acids encoding the L chains, to produce an antibody library containing the H chains and L chains of the antibodies that recognize the second antigen, and
      (2-B) a step of selecting the antibodies that recognize the second antigen from the antibody library obtained in the step (2-A).
< 10 > The method according to < 1 > above, wherein the first antigen and the second antigen are each selected from a cancer antigen, an immune cell antigen, a bioactive molecule, and an enzyme.
< 11 > A method for producing a multispecific antibody, comprising:
   a step of identifying an L chain by the method according to any one of < 1 > to < 10 > above, and
   a step of producing a multispecific antibody, using the L chain identified above, the H chain recognizing the first antigen, and the H chain recognizing the second antigen.

### Advantageous Effects of Invention

According to the present invention, a common L chain for use in production of a multispecific antibody can be identified. By commonizing multiple L chains necessary for production of a multispecific antibody, the number of components of the multispecific antibody can be reduced, and the steps involved in the expression and purification of the multispecific antibody can be reduced.

### Brief Description of Drawings

[Figure 1] Figure 1 shows a first aspect of the present invention.
[Figure 2] Figure 2 shows a second aspect of the present invention.
[Figure 3] Figure 3 shows a third aspect of the present invention.
[Figure 4] Figure 4 shows the results of identification of a common light chain sequence.
[Figure 5] Figure 5 shows the results of identification of a common light chain sequence.
[Figure 6] Figure 6 shows the results obtained by confirming reactivity by performing ELISA on the results obtained by screening from an L chain library, into which the H chain of Cetuximab has been inserted.
[Figure 7] Figure 7 shows the results obtained by confirming reactivity by performing ELISA on the results obtained by screening from an L chain library, into which the H chain of Panitumumab has been inserted.
[Figure 8] Figure 8 shows the results obtained by confirming ELISA reactivity to a TfR protein.

### Embodiments of Carrying out the Invention

Next, the present invention will be described in more detail.

The present invention relates to a method for identifying a common L chain for use in production of a multispecific antibody, comprising:
(1) a step of producing a first library containing multiple antibodies that recognize a first antigen,
(2) a step of producing a second library containing multiple antibodies that recognize a second antigen, and
(3) a step of selecting an antibody that recognizes the first antigen and an antibody that recognizes the second antigen, which have a common L chain, from the first library and the second library.

The multispecific antibody is an antibody that recognizes two or more types of antigens. In particular, a bispecific antibody is commonly used.

The types of the first and second antigens recognized by the multispecific antibody are not particularly limited, and for example, the first and second antigens can be each selected from a cancer antigen, an immune cell antigen, a bioactive molecule, and an enzyme. For example, combinations of the first and second antigens include, but are not particularly limited to, a combination of a cancer antigen and a cancer antigen, a combination of a cancer antigen and an immune cell antigen, a combination of a bioactive molecule and a bioactive molecule, and a combination of an enzyme and an enzyme.

Examples of the cancer antigen may include CD19, CD20, CD33, CD38, CD123, CD135, EpCAM, CEA, CDH3, Her2, HLAA/gp100, PSMA, BCMA, MAGE-A4, PRAME, GPC3, GD2, EGFR, MUC1, MUC16, MUC17, MSLN, EphA2, ROR1, and DLL3.

Examples of the immune cell antigen may include CD3, CD47, and OX40.

Examples of the bioactive molecule may include cytokines, hormones, and neurotransmitters. Specific examples thereof may include IL-2, IFN-α, -β and -γ, TNF, HMGB1, and HMGB2.

Examples of the enzyme may include CD73, CD39, and MMPs such as MMP-2 and MMP-9.

The library containing antibodies is not particularly limited, and a library in which single-chain antibodies (scFv) are displayed on the surface of phages by a phage display method, a library using a cell-free translation system, a library in which antibodies are displayed on the surface of cells or viruses, a library using emulsions, etc. are known. For example, the techniques using cell-free translation systems include a ribosome display method, which forms a complex of mRNA and a translated protein via ribosomes by the removal, etc. of stop codons, a cDNA display method, which covalently binds a gene sequence and a translated protein using compounds such as puromycin, an mRNA display method, and a CIS display method, which forms a complex of a gene and a translated protein using a nucleic acid-binding protein. The techniques of displaying antigen-binding molecules on the surface of cells or viruses include, in addition to the phage display method, an *E. coli* display method, a Gram-positive bacterium display method, a yeast display method, a mammalian cell display method, and a virus display method. The techniques using emulsions include an *vitro* virus display method comprising encapsulating a gene and a translation-related molecule in an emulsion.

An antibody that recognizes a specific antigen can be selected using a known method. Examples of such a known method may include enzyme-linked immunosorbent assay (ELISA), electrochemiluminescence (ECL), fluorescence-activated cell sorting (FACS), and a method utilizing surface plasmon resonance (SPR).

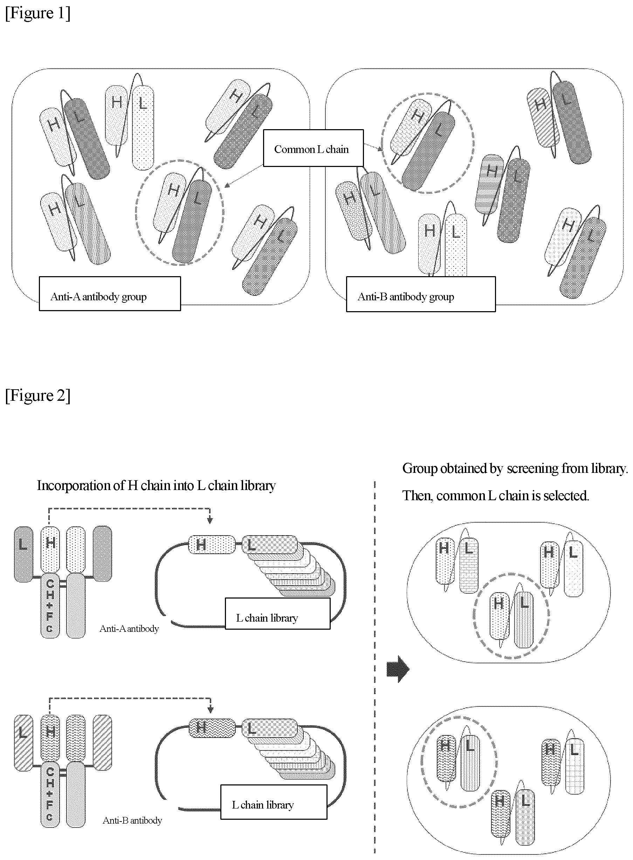

According to a first aspect of the present invention, the step (1) of producing a first library containing multiple antibodies that recognize a first antigen is a step of selecting multiple antibodies that recognize a first antigen from a library containing multiple antibodies having H chains and L chains, so as to produce a first library, and the step (2) of producing a second library containing multiple antibodies that recognize a second antigen is a step of selecting multiple antibodies that recognize a second antigen from a library containing multiple antibodies having H chains and L chains, so as to produce a second library. Thereafter, an antibody that recognizes the first antigen and an antibody that recognizes the second antigen, which have a common L chain, are selected from among the above-described first library and the above-described second library, so that the common L chain can be identified. The first aspect of the present invention is shown in Figure 1. With respect to Antigen A (the first antigen) and antigen B (the second antigen), screening is performed from the antibody library using the phage display method. By performing this screening, multiple clones that have binding ability to each antigen are obtained. From the obtained anti-antigen A antibody group and anti-antigen B antibody group, clones with a common L chain are selected.

In the first aspect of the present invention, the library containing multiple antibodies having H chains and L chains preferably comprises 10⁹ types or more of antibodies, more preferably comprises 2 x 10⁹ types or more of antibodies, and further preferably comprises 5 x 10⁹ types or more of antibodies.

The types of H chains in the library containing multiple antibodies having H chains and L chains is preferably 10⁶ or more, more preferably 2 x 10⁶ or more, and further preferably 5 x 10⁶ or more.

The types of L chains in the library containing multiple antibodies having H chains and L chains is preferably 10³ or less, more preferably 700 or less, and further preferably 500 or less.

Moreover, the ratio between the types of H chains and the types of L chains in the library containing multiple antibodies having H chains and L chains is preferably 1000 times or more, more preferably 5000 times or more, and further preferably 10000 times or more.

As described above, by using a library in which the types of L chains are limited, the likelihood of obtaining L chains that also react with the antigen A (first antigen) and the antigen B (second antigen) increases.

According to a second aspect of the present invention,
the step (1) comprises:
   (1-A) a step of incorporating nucleic acids encoding the H chains of the antibodies that recognize a first antigen into a library of nucleic acids encoding the L chains, to produce an antibody library containing the H chains and L chains of the antibodies that recognize the first antigen, and
   (1-B) a step of selecting the antibodies that recognize the first antigen from the antibody library obtained in the step (1-A), and
the step (2) comprises:
   (2-A) a step of incorporating nucleic acids encoding the H chains of the antibodies that recognize a second antigen into a library of nucleic acids encoding the L chains, to produce an antibody library containing the H chains and L chains of the antibodies that recognize the second antigen, and
   (2-B) a step of selecting the antibodies that recognize the second antigen from the antibody library obtained in the step (2-A). The second aspect of the present invention is shown in Figure 2. In the second aspect, the H chain portion of the anti-antigen A antibody is introduced into the L chain library. Similarly, the H chain portion of the anti-antigen B antibody is introduced into the L chain library. The respective antibody libraries are screened for the antigen A (first antigen) and the antigen B (second antigen) using the phage display method. By performing the screening, multiple clones having binding ability to each antigen are obtained. From the obtained anti-antigen A antibody group and anti-antigen B antibody group, clones having a common L chain are selected.

In the second aspect of the present invention, the types of L chains in the antibody library obtained in the step (1-A) and the antibody library obtained in the step (2-A), respectively, are preferably 10³ or less, more preferably 700 or less, and further preferably 500 or less.

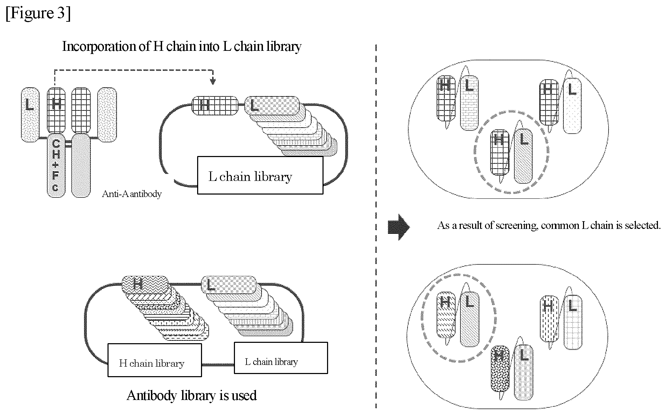

According to a third aspect of the present invention,
the step (1) is a step of producing a first library containing multiple antibodies that recognize a first antigen is a step of selecting multiple antibodies that recognize a first antigen from a library containing multiple antibodies having H chains and L chains, so as to produce a first library, and
the step (2) comprises:
   (2-A) a step of incorporating nucleic acids encoding the H chains of the antibodies that recognize a second antigen into a library of nucleic acids encoding the L chains, to produce an antibody library containing the H chains and L chains of the antibodies that recognize the second antigen, and
   (2-B) a step of selecting the antibodies that recognize the second antigen from the antibody library obtained in the step (2-A). The third aspect of the present invention is shown in Figure 3.

The method for producing a library of L chains (light chains) used in the present invention is not particularly limited.

The germline genes of human antibodies are publicly available on "THE INTERNATIONAL IMMUNOGENETICS INFORMATION SYSTEM (IMGT: https://www.imgt.org/)". Upon examining the subgroups of the light chain variable region, the kappa chain comprises 35 to 40 functional clusters of IGKV (IGKV1: 17 (+2) types, IGKV2: 9 (+1) types, IGKV3: 5 (+2) types, IGKV4: 1 type, IGKV5: 5 types, IGKV6: 2 types, and IGKV7: - type), whereas the lambda chain comprises 29 or 30 types of functional clusters of IGLV (IGLV1: 5 types, IGLV2: 5 types, IGLV3: 8 types, IGLV4: 3 types, IGLV5: 3-4 types, IGLV6: 1 type, IGLV7: 1 type, IGLV8: 1 type, IGLV9: 1 type, IGLV10: 1 species, and IGLV11: - type) (Williams, S. C. et al. J. Mol. Biol., 264, 220-232 (1996), Kawasaki, K. et al. Genome Res., 7, 250-261 (1997)).

Referring to the above, the sequences shown in Table 1 below (SEQ ID NOs: 1 to 39) can be designed as basic germline amino acid sequences for the kappa chain, and the sequences shown in Table 2 below (SEQ ID NOs: 40 to 70) can be designed as basic germline amino acid sequences for the lambda chain. The sequences included in the kappa chain basic germline amino acid sequences and the lambda chain basic germline amino acid sequences described above are the V region, but the J region and CL region are not included. Therefore, the light chain J region can be assigned to the light chain variable region by referring to the human J gene sequences (SEQ ID NOs: 71 to 83 listed in Tables 3 and 4), and the light chain constant region can be assigned to the light chain variable region by referring to the CL gene sequence (SEQ ID NOs: 84 and 85).

Furthermore, in order to impart diversity to the light chain basic sequences described above, the proportion of family gene subgroups can be adjusted to match the proportion present in the living human body, and further, mutations or additional sequences can be artificially inserted into the frame regions FR1, FR2, FR3, and FR4 and the complementarity-determining regions CDR1, CDR2, and CDR3. Specifically, with regard to the proportions of the kappa and lambda chain gene families present in the living body, the proportions of individual subgroups present in the living body can be determined by referring to previous studies, etc. (Griffiths AD, et al. (1994), Prabakaran P, et al. (2012), etc.). For example, among the kappa chains (IGKV1 to IGKV6), the proportions of individual subgroups in the library can be approximately set as follows: IGKV1: 36%, IGKV2: 11%, IGKV3: 35%, IGKV4: 11%, IGKV5: 4%, and IGKV6: 3%. Among the lambda chains (IGLV1 to IGLV10), the proportions of individual subgroups in the library can be approximately set as follows: IGLV1: 40%, IGLV2: 16%, IGLV3: 21%, IGLV4: 3%, IGLV5: 6%, IGLV6: 6%, IGLV7: 2%, IGLV8: 2%, IGLV9: 2%, and IGLV10: 2%. Regarding CDR3, which is thought to be most involved in antigen recognition, the length can be set as 7 to 11 amino acids wide for the kappa chain, with an average of 9.2 amino acids, and 8 to 12 amino acids wide for the lambda chain, with an average of 10.4 amino acids. The GRAVY index (Grand Average of Hydropathicity), an indicator of hydrophobicity, can be set as an average of -1.00 for the kappa chain, and as an average of -0.38 for the lambda chain. Furthermore, the number of non-polar amino acids (A, C, F, G, I, L, M, P, V, W, and Y) can be designed to be an average of 3.9 for the kappa chain and an average of 4.7 for the lambda chain.

As described above, it is preferable to use a library of L chains (light chains) used in the present invention, which is characterized in that the library reflects the naive repertoire, in that the L chain family genes are set to match the proportions in vivo, and in that artificial mutations have been inserted therein.

According to the present invention,
provided is a method for producing a multispecific antibody, comprising:
a step of identifying an L chain by the method of the present invention, and
a step of producing a multispecific antibody, using the L chain identified above, the H chain recognizing the first antigen, and the H chain recognizing the second antigen.

In the present invention, since a multispecific antibody is produced using a common L chain, the number of L chain and H chain combinations is reduced, making the expression and purification of the multispecific antibody easier.

In the step of producing a multispecific antibody using an L chain, an H chain that recognizes a first antigen, and an H chain that recognizes a second antigen, the DNAs encoding the L chain, the H chain that recognizes the first antigen, and the H chain that recognizes the second antigen, respectively, are inserted into a vector to prepare an expression vector, and the expression vector is then allowed to express in host cells, and the antibody can be obtained by recovering and purifying the secreted supernatant. In order to produce a bispecific antibody, which contains two pairs of different H chain-L chain pairs (an H chain-L chain pair that recognizes the first antigen and an H chain-L chain pair that recognizes the second antigen), it is necessary to distinguish an antibody consisting of two identical pairs of an H chain and an L chain that recognize the first antigen from an antibody consisting of two identical pairs of an H chain and an L chain that recognize the second antigen, and then to purify them separately. Even using a common L chain, this distinction and purification are still necessary, but techniques for efficiently producing bispecific antibodies have been established through the previous study ('Knobs-into-holes' Protein Eng. 1996 Jul; 9(7): 617-21.), etc. From these findings, by combining the 'knobs-into-holes' technique with antibody design using the common L chain can improve expression and purification efficiency.

Examples of the expression vector may include plasmids, retroviruses, adenoviruses, adeno-associated viruses (AAVs), plant viruses such as cauliflower mosaic virus and tobacco mosaic virus, cosmids, YACs, and EBV-derived episomes. The expression vector and the expression regulatory sequence are selected to be compatible with the host cells used for expression. An antibody light chain gene and an antibody heavy chain gene can be inserted into separate vectors, or both genes can be inserted into the same expression vector. The antibody gene is inserted into the expression vector using standard methods (e.g., ligation of complementary restriction sites on the antibody gene fragment to the vector, or blunt-end ligation if no restriction sites exist).

As described above, a suitable vector encodes a functionally complete human CH or CL immunoglobulin sequence having appropriate restriction sites, which are engineered to allow for the easy insertion and expression of any VH or VL sequence. In such a vector, in general, splicing occurs between the splice donor site in the inserted J region and the splice acceptor site preceding the human C domain, as well as in splice regions located within the human CH exon. Polyadenylation and transcription termination occur at native chromosomal sites downstream of the coding region. Recombinant expression vectors can also encode signal peptides that promote secretion of antibody chains derived from host cells. The antibody chain gene can be cloned into the vector, so that the signal peptide is in-frame ligated to the amino terminus of the immunoglobulin chain. The signal peptide may be either an immunoglobulin signal peptide or a heterologous signal peptide (i.e., a signal peptide derived from a non-immunoglobulin protein).

The antibody expression vector may also have, in addition to the antibody gene and the regulatory sequence, further sequences such as sequences that control vector replication in host cells (e.g., origin of replication) and selection marker genes. Such selection marker genes promote selection of host cells, into which the vector has been introduced. For example, selection marker genes generally confer resistance to drugs such as G418, hygromycin or methotrexate, to host cells into which the vector has been introduced. Preferred selection marker genes include the dihydrofolate reductase (DHFR) gene (used in dhfr-host cells along with methotrexate for selection/amplification), the neomycin phosphotransferase gene (for G418 selection), and the glutamate synthase gene.

The host cells are transformed with the antibody gene expression vector prepared by the above method. The host cells can be any cells capable of producing antibodies, such as bacteria, yeasts, animal cells, insect cells, or plant cells, with animal cells being preferable. Examples of the animal cells may include Chinese hamster ovary cells such as CHO/dhfr (-) cells and CHO/DG44 cells, monkey-derived COS cells (A. Wright & S. L. Morrison, J. Immunol. 160, 3393-3402 (1998)), and SP2/O cells (mouse myeloma) (K. Motmans et al., Eur. J. Cancer Prev. 5, 512-5199 (1996), R. P. Junghans et al., Cancer Res. 50, 1495-1502 (1990)). In addition, for transformation, a lipofectin method (R. W. Malone et al., Proc. Natl. Acad. Sci. USA 86, 6007 (1989), P. L. Felgner et al., Proc. Natl. Acad. Sci. USA 84, 7413 (1987)), an electroporation method, a calcium phosphate method (F. L. Graham & A. J. van der Eb, Virology 52, 456-467 (1973)), a DEAE-Dextran method, etc. are preferably used.

After culturing the transformant, an antibody is isolated from the cells or culture solution of the transformant. For isolation and purification of the antibody, methods such as centrifugation, ammonium sulfate fractionation, salting out, ultrafiltration, affinity chromatography, ion exchange chromatography, and gel filtration chromatography, can be combined as appropriate, and can be utilized.

### < Some examples of antibodies >

According to the present invention, a CD3ε antibody obtained in the embodiments described below is provided. Specifically, according to the present invention, provided is an antibody comprising a heavy chain variable region consisting of the amino acid sequence of SEQ ID NO: 97 and a light chain variable region consisting of the amino acid sequence of SEQ ID NO: 99. Furthermore, according to the present invention, provided is an antibody comprising a heavy chain variable region consisting of the amino acid sequence of SEQ ID NO: 97 and a light chain consisting of the amino acid sequence of SEQ ID NO: 98.
SEQ ID NO: 97: CD3ε antibody _0039 heavy chain variable region 
QVQLVQSGAEVKKPGASVKVSCKASGYTFTGYYMHWVRQAPGQGLEWMGWINPNSG
GTNYAQKFQGRVTMTRDTSISTAYMELSRLRSDDTAVYYCAREMSNWDDAFDIWGQGT
MVTVSR
SEQ ID NO: 98: CD3ε antibody _0039 light chain 
SYELTQPPSVSVAPGKTARITCGGNNIGSKSVHWYQQKPGQAPVLVVYDDSDRPSGIPER
FSGSNSGNTATLTISRVEAGDEADYYCQVWDSSSDHYVFGTGTKVTVLGQPKANPTVTL
FPPSSEELQANKATLVCLISDFYPGAVTVAWKADGSPVKAGVETTKPSKQSNNKYAASS
YLSLTPEQWKSHRSYSCQVTHEGSTVEKTVAPTECS
 CD3 light chain variable region sequence added
SEQ ID NO: 99: CD3ε antibody _0039 light chain variable region

SYELTQPPSVSVAPGKTARITCGGNNIGSKSVHWYQQKPGQAPVLVVYDDSDRPSGIPER
FSGSNSGNTATLTISRVEAGDEADYYCQVWDSSSDHYVFGTGTKVTVLG

Hereinafter, the present invention will be described in the following examples. However, the present invention is not limited by these examples.

### Examples

### < Preparation of light chain library >

### (1) Design of light chains

The germline genes of human antibodies are publicly available on "THE INTERNATIONAL IMMUNOGENETICS INFORMATION SYSTEM (IMGT: https://www.imgt.org/)". Upon examining the subgroups of the light chain variable region, the kappa chain comprises 35 to 40 functional clusters of IGKV (IGKV1: 17 (+2) types, IGKV2: 9 (+1) types, IGKV3: 5 (+2) types, IGKV4: 1 type, IGKV5: 5 types, IGKV6: 2 types, and IGKV7: - type), whereas the lambda chain comprises 29 or 30 types of functional clusters of IGLV (IGLV1: 5 types, IGLV2: 5 types, IGLV3: 8 types, IGLV4: 3 types, IGLV5: 3-4 types, IGLV6: 1 type, IGLV7: 1 type, IGLV8: 1 type, IGLV9: 1 type, IGLV10: 1 species, and IGLV11: - type) (Williams, S. C. et al. J. Mol. Biol., 264, 220-232 (1996), Kawasaki, K. et al. Genome Res., 7, 250-261 (1997)).

Thus, referring to this, basic germline genes were designed (Table 1 and Table 2).

**[Table 1]**

| (Table 1) Kappa chain basic germline amino acid sequences | |
|---|---|
| | |
| IGKV1-5 | DIQMTQSPSTLSASVGDRVTITCRASQSISSWLAWYQQKPGKAPKLLIYDAS SLESGVPSRFSGSGSGTEFTLTISSLQPDDFATYYCQQYNSYS (SEQ ID NO: 1) |
| IGKV1-6 | AIQMTQSPSSLSASVGDRVTITCRASQGIRNDLGWYQQKPGKAPKLLIYAA SSLQSGVPSRFSGSGSGTDFTLTISSLQPEDFATYYCLQDYNYP (SEQ ID NO: 2) |
| IGKV1-8 | AIRMTQSPSSFSASTGDRVTITCRASQGISSYLAWYQQKPGKAPKLLIYAAS TLQSGVPSRFSGSGSGTDFTLTISCLQSEDFATYYCQQYYSYP (SEQ ID NO: 3) |
| IGKV1-9 | DIQLTQSPSFLSASVGDRVTITCRASQGISSYLAWYQQKPGKAPKLLIYAAST LQSGVPSRFSGSGSGTEFTLTISSLQPEDFATYYCQQLNSYP (SEQ ID NO: 4) |
| IGKV1-12 | DIQMTQSPSSVSASVGDRVTITCRASQGISSWLAWYQQKPGKAPKLLIYAA SSLQSGVPSRFSGSGSGTDFTLTISSLQPEDFATYYCQQANSFP (SEQ ID NO: 5) |
| IGKV1-16 | DIQMTQSPSSLSASVGDRVTITCRASQGISNYLAWFQQKPGKAPKSLIYAAS SLQSGVPSRFSGSGSGTDFTLTISSLQPEDFATYYCQQYNSYP (SEQ ID NO: 6) |
| IGKV1-17 | DIQMTQSPSSLSASVGDRVTITCRASQGIRNDLGWYQQKPGKAPKRLIYAA SSLQSGVPSRFSGSGSGTEFTLTISSLQPEDFATYYCLQHNSYP (SEQ ID NO: 7) |
| IGKV1-27 | DIQMTQSPSSLSASVGDRVTITCRASQGISNYLAWYQQKPGKVPKLLIYAAS TLQSGVPSRFSGSGSGTDFTLTISSLQPEDVATYYCQKYNSAP (SEQ ID NO: 8) |
| IGKV1-33 | DIQMTQSPSSLSASVGDRVTITCQASQDISNYLNWYQQKPGKAPKLLIYDA SNLETGVPSRFSGSGSGTDFTFTISSLQPEDIATYYCQQYDNLP (SEQ ID NO: 9) |
| IGKV1-39 | DIQMTQSPSSLSASVGDRVTITCRASQSISSYLNWYQQKPGKAPKLLIYAAS SLQSGVPSRFSGSGSGTDFTLTISSLQPEDFATYYCQQSYSTP (SEQ ID NO: 10) |
| IGKV1D-8 | VIWMTQSPSLLSASTGDRVTISCRMSQGISSYLAWYQQKPGKAPELLIYAAS TLQSGVPSRFSGSGSGTDFTLTISCLQSEDFATYYCQQYYSFP (SEQ ID NO: 11) |
| IGKV1D-12 | DIQMTQSPSSVSASVGDRVTITCRASQGISSWLAWYQQKPGKAPKLLIYAA SSLQSGVPSRFSGSGSGTDFTLTISSLQPEDFATYYCQQANSFP (SEQ ID NO: 12) |
| IGKV1D-13 | AIQLTQSPSSLSASVGDRVTITCRASQGISSALAWYQQKPGKAPKLLIYDAS SLESGVPSRFSGSGSGTDFTLTISSLQPEDFATYYCQQFNNYP (SEQ ID NO: 13) |
| IGKV1D-16 | DIQMTQSPSSLSASVGDRVTITCRASQGISSWLAWYQQKPEKAPKSLIYAAS SLQSGVPSRFSGSGSGTDFTLTISSLQPEDFATYYCQQYNSYP (SEQ ID NO: 14) |
| IGKV1D-17 | NIQMTQSPSAMSASVGDRVTITCRARQGISNYLAWFQQKPGKVPKHLIYAA SSLQSGVPSRFSGSGSGTEFTLTISSLQPEDFATYYCLQHNSYP (SEQ ID NO: 15) |
| IGKV1D-33 | DIQMTQSPSSLSASVGDRVTITCQASQDISNYLNWYQQKPGKAPKLLIYDA SNLETGVPSRFSGSGSGTDFTFTISSLQPEDIATYYCQQYDNLP (SEQ ID NO: 16) |
| IGKV1D-39 | DIQMTQSPSSLSASVGDRVTITCRASQSISSYLNWYQQKPGKAPKLLIYAAS SLQSGVPSRFSGSGSGTDFTLTISSLQPEDFATYYCQQSYSTP (SEQ ID NO: |
| | 17) |
| IGKV1D-43 | AIRMTQSPFSLSASVGDRVTITCWASQGISSYLAWYQQKPAKAPKLFIYYAS SLQSGVPSRFSGSGSGTDYTLTISSLQPEDFATYYCQQYYSTP (SEQ ID NO: 18) |
| IGKV1-NL1 | DIQMTQSPSSLSASVGDRVTITCRASQGISNSLAWYQQKPGKAPKLLLYAAS RLESGVPSRFSGSGSGTDYTLTISSLQPEDFATYYCQQYYSTP (SEQ ID NO: 19) |
| IGKV2-24 | DIVMTQTPLSSPVTLGQPASISCRSSQSLVHSDGNTYLSWLQQRPGQPPRLLI YKISNRFSGVPDRFSGSGAGTDFTLKISRVEAEDVGVYYCMQATQFP (SEQ ID NO: 20) |
| IGKV2-28 | DIVMTQSPLSLPVTPGEPASISCRSSQSLLHSNGYNYLDWYLQKPGQSPQLL IYLGSNRASGVPDRFSGSGSGTDFTLKISRVEAEDVGVYYCMQALQTP (SEQ ID NO: 21) |
| IGKV2-30 | DVVMTQSPLSLPVTLGQPASISCRSSQSLVYSDGNTYLNWFQQRPGQSPRR LIYKVSNRDSGVPDRFSGSGSGTDFTLKISRVEAEDVGVYYCMQGTHWP (SEQ ID NO: 22) |
| IGKV2-40 | DIVMTQTPLSLPVTPGEPASISCRSSQSLLDSDDGNTYLDWYLQKPGQSPQL LIYTLSYRASGVPDRFSGSGSGTDFTLKISRVEAEDVGVYYCMQRIEFP (SEQ ID NO: 23) |
| IGKV2D-26 | EIVMTQTPLSLSITPGEQASISCRSSQSLLHSDGYTYLYWFLQKARPVSTLLI YEVSNRFSGVPDRFSGSGSGTDFTLKISRVEAEDFGVYYCMQDAQDP (SEQ ID NO: 24) |
| IGKV2D-28 | DIVMTQSPLSLPVTPGEPASISCRSSQSLLHSNGYNYLDWYLQKPGQSPQLL IYLGSNRASGVPDRFSGSGSGTDFTLKISRVEAEDVGVYYCMQALQTP (SEQ ID NO: 25) |
| IGKV2D-29 | DIVMTQTPLSLSVTPGQPASISCKSSQSLLHSDGKTYLYWYLQKPGQPPQLL IYEVSNRFSGVPDRFSGSGSGTDFTLKISRVEAEDVGVYYCMQSIQLP (SEQ ID NO: 26) |
| IGKV2D-30 | DVVMTQSPLSLPVTLGQPASISCRSSQSLVYSDGNTYLNWFQQRPGQSPRR LIYKVSNWDSGVPDRFSGSGSGTDFTLKISRVEAEDVGVYYCMQGTHWP (SEQ ID NO: 27) |
| IGKV2D-40 | DIVMTQTPLSLPVTPGEPASISCRSSQSLLDSDDGNTYLDWYLQKPGQSPQL LIYTLSYRASGVPDRFSGSGSGTDFTLKISRVEAEDVGVYYCMQRIEFP (SEQ ID NO: 28) |
| IGKV3-11 | EIVLTQSPATLSLSPGERATLSCRASQSVSSYLAWYQQKPGQAPRLLIYDAS NRATGIPARFSGSGSGTDFTLTISSLEPEDFAVYYCQQRSNWP (SEQ ID NO: 29) |
| IGKV3-15 | EIVMTQSPATLSVSPGERATLSCRASQSVSSNLAWYQQKPGQAPRLLIYGAS TRATGIPARFSGSGSGTEFTLTISSLQSEDFAVYYCQQYNNWP (SEQ ID NO: 30) |
| IGKV3-20 | EIVLTQSPGTLSLSPGERATLSCRASQSVSSSYLAWYQQKPGQAPRLLIYGA SSRATGIPDRFSGSGSGTDFTLTISRLEPEDFAVYYCQQYGSSP (SEQ ID NO: 31) |
| IGKV3D-7 | EIVMTQSPATLSLSPGERATLSCRASQSVSSSYLSWYQQKPGQAPRLLIYGA STRATGIPARFSGSGSGTDFTLTISSLQPEDFAVYYCQQDYNLP (SEQ ID NO: 32) |
| IGKV3D-11 | EIVLTQSPATLSLSPGERATLSCRASQGVSSYLAWYQQKPGQAPRLLIYDAS NRATGIPARFSGSGPGTDFTLTISSLEPEDFAVYYCQQRSNWH (SEQ ID NO: 33) |
| IGKV3D-15 | EIVMTQSPATLSVSPGERATLSCRASQSVSSNLAWYQQKPGQAPRLLIYGAS TRATGIPARFSGSGSGTEFTLTISSLQSEDFAVYYCQQYNNWP (SEQ ID NO: 34) |
| IGKV3D-20 | EIVLTQSPATLSLSPGERATLSCGASQSVSSSYLAWYQQKPGLAPRLLIYDAS SRATGIPDRFSGSGSGTDFTLTISRLEPEDFAVYYCQQYGSSP (SEQ ID NO: 35) |
| IGKV4-1 | DIVMTQSPDSLAVSLGERATINCKSSQSVLYSSNNKNYLAWYQQKPGQPPK LLIYWASTRESGVPDRFSGSGSGTDFTLTISSLQAEDVAVYYCQQYYSTP (SEQ ID NO: 36) |
| IGKV5-2 | ETTLTQSPAFMSATPGDKVNISCKASQDIDDDMNWYQQKPGEAAIFIIQEAT TLVPGIPPRFSGSGYGTDFTLTINNIESEDAAYYFCLQHDNFP (SEQ ID NO: 37) |
| IGKV6-21 | EIVLTQSPDFQSVTPKEKVTITCRASQSIGSSLHWYQQKPDQSPKLLIKYASQ SFSGVPSRFSGSGSGTDFTLTINSLEAEDAATYYCHQSSSLP (SEQ ID NO: 38) |
| IGKV6D-21 | EIVLTQSPDFQSVTPKEKVTITCRASQSIGSSLHWYQQKPDQSPKLLIKYASQ SFSGVPSRFSGSGSGTDFTLTINSLEAEDAATYYCHQSSSLP (SEQ ID NO: 39) |

**[Table 2]**

| (Table 2) Lambda chain basic germline amino acid sequences | |
|---|---|
| IGLV1-36 | QSVLTQPPSVSEAPRQRVTISCSGSSSNIGNNAVNWYQQLPGKAPKLLIYYD DLLPSGVSDRFSGSKSGTSASLAISGLQSEDEADYYCAAWDDSLNG (SEQ ID NO: 40) |
| IGLV1-40 | QSVLTQPPSVSGAPGQRVTISCTGSSSNIGAGYDVHWYQQLPGTAPKLLIYG NSNRPSGVPDRFSGSKSGTSASLAITGLQAEDEADYYCQSYDSSLSG (SEQ ID NO: 41) |
| IGLV1-44 | QSVLTQPPSASGTPGQRVTISCSGSSSNIGSNTVNWYQQLPGTAPKLLIYSN NQRPSGVPDRFSGSKSGTSASLAISGLQSEDEADYYCAAWDDSLNG (SEQ ID NO: 42) |
| IGLV1-47 | QSVLTQPPSASGTPGQRVTISCSGSSSNIGSNYVYWYQQLPGTAPKLLIYRN NQRPSGVPDRFSGSKSGTSASLAISGLRSEDEADYYCAAWDDSLSG (SEQ ID NO: 43) |
| IGLV1-51 | QSVLTQPPSVSAAPGQKVTISCSGSSSNIGNNYVSWYQQLPGTAPKLLIYDN NKRPSGIPDRFSGSKSGTSATLGITGLQTGDEADYYCGTWDSSLSA (SEQ ID NO: 44) |
| IGLV2-8 | QSALTQPPSASGSPGQSVTISCTGTSSDVGGYNYVSWYQQHPGKAPKLMIY EVSKRPSGVPDRFSGSKSGNTASLTVSGLQAEDEADYYCSSYAGSNNF (SEQ ID NO: 45) |
| IGLV2-11 | QSALTQPRSVSGSPGQSVTISCTGTSSDVGGYNYVSWYQQHPGKAPKLMI YDVSKRPSGVPDRFSGSKSGNTASLTISGLQAEDEADYYCCSYAGSYTF (SEQ ID NO: 46) |
| IGLV2-14 | QSALTQPASVSGSPGQSITISCTGTSSDVGGYNYVSWYQQHPGKAPKLMIY EVSNRPSGVSNRFSGSKSGNTASLTISGLQAEDEADYYCSSYTSSSTL (SEQ ID NO: 47) |
| IGLV2-18 | QSALTQPPSVSGSPGQSVTISCTGTSSDVGSYNRVSWYQQPPGTAPKLMIYE VSNRPSGVPDRFSGSKSGNTASLTISGLQAEDEADYYCSLYTSSSTF (SEQ ID NO: 48) |
| IGLV2-23 | QSALTQPASVSGSPGQSITISCTGTSSDVGSYNLVSWYQQHPGKAPKLMIYE GSKRPSGVSNRFSGSKSGNTASLTISGLQAEDEADYYCCSYAGSSTL (SEQ ID NO: 49) |
| IGLV3-1 | SYELTQPPSVSVSPGQTASITCSGDKLGDKYACWYQQKPGQSPVLVIYQDS KRPSGIPERFSGSNSGNTATLTISGTQAMDEADYYCQAWDSSTA (SEQ ID NO: 50) |
| IGLV3-9 | SYELTQPLSVSVALGQTARITCGGNNIGSKNVHWYQQKPGQAPVLVIYRDS |
| | NRPSGIPERFSGSNSGNTATLTISRAQAGDEADYYCQVWDSSTA (SEQ ID NO: 51) |
| IGLV3-10 | SYELTQPPSVSVSPGQTARITCSGDALPKKYAYWYQQKSGQAPVLVIYEDS KRPSGIPERFSGSSSGTMATLTISGAQVEDEADYYCYSTDSSGNH (SEQ ID NO: 52) |
| IGLV3-16 | SYELTQPPSVSVSLGQMARITCSGEALPKKYAYWYQQKPGQFPVLVIYKDS ERPSGIPERFSGSSSGTIVTLTISGVQAEDEADYYCLSADSSGTY (SEQ ID NO: 53) |
| IGLV3-19 | SSELTQDPAVSVALGQTVRITCQGDSLRSYYASWYQQKPGQAPVLVIYGKN NRPSGIPDRFSGSSSGNTASLTITGAQAEDEADYYCNSRDSSGNH (SEQ ID NO: 54) |
| IGLV3-21 | SYVLTQPPSVSVAPGKTARITCGGNNIGSKSVHWYQQKPGQAPVLVIYYDS DRPSGIPERFSGSNSGNTATLTISRVEAGDEADYYCQVWDSSSDH (SEQ ID NO: 55) |
| IGLV3-22 | SYELTQLPSVSVSPGQTARITCSGDVLGENYADWYQQKPGQAPELVIYEDS ERYPGIPERFSGSTSGNTTTLTISRVLTEDEADYYCLSGDEDN (SEQ ID NO: 56) |
| IGLV3-25 | SYELMQPPSVSVSPGQTARITCSGDALPKQYAYWYQQKPGQAPVLVIYKDS ERPSGIPERFSGSSSGTTVTLTISGVQAEDEADYYCQSADSSGTY (SEQ ID NO: 57) |
| IGLV3-27 | SYELTQPSSVSVSPGQTARITCSGDVLAKKYARWFQQKPGQAPVLVIYKDS ERPSGIPERFSGSSSGTTVTLTISGAQVEDEADYYCYSAADNN (SEQ ID NO: 58) |
| IGLV4-60 | QPVLTQSSSASASLGSSVKLTCTLSSGHSSYIIAWHQQQPGKAPRYLMKLEG SGSYNKGSGVPDRFSGSSSGADRYLTISNLQLEDEADYYCETWDSNT (SEQ ID NO: 59) |
| IGLV4-69 | QLVLTQSPSASASLGASVKLTCTLSSGHSSYAIAWHQQQPEKGPRYLMKLN SDGSHSKGDGIPDRFSGSSSGAERYLTISSLQSEDEADYYCQTWGTGI (SEQ ID NO: 60) |
| IGLV5-37 | QPVLTQPPSSSASPGESARLTCTLPSDINVGSYNIYWYQQKPGSPPRYLLYY YSDSDKGQGSGVPSRFSGSKDASANTGILLISGLQSEDEADYYCMIWPSNA S (SEQ ID NO: 61) |
| IGLV5-39 | QPVLTQPTSLSASPGASARFTCTLRSGINVGTYRIYWYQQKPGSLPRYLLRY KSDSDKQQGSGVPSRFSGSKDASTNAGLLLISGLQSEDEADYYCAIWYSST |
| | S (SEQ ID NO: 62) |
| IGLV5-45 | QAVLTQPASLSASPGASASLTCTLRSGINVGTYRIYWYQQKPGSPPQYLLRY KSDSDKQQGSGVPSRFSGSKDASANAGILLISGLQSEDEADYYCMIWHSSA S (SEQ ID NO: 63) |
| IGLV5-52 | QPVLTQPSSHSASSGASVRLTCMLSSGFSVGDFWIRWYQQKPGNPPRYLLY YHSDSNKGQGSGVPSRFSGSNDASANAGILRISGLQPEDEADYYCGTWHS NSKT (SEQ ID NO: 64) |
| IGLV6-57 | NFMLTQPHSVSESPGKTVTISCTRSSGSIASNYVQWYQQRPGSSPTTVIYED NQRPSGVPDRFSGSIDSSSNSASLTISGLKTEDEADYYCQSYDSSN (SEQ ID NO: 65) |
| IGLV7-43 | QTVVTQEPSLTVSPGGTVTLTCASSTGAVTSGYYPNWFQQKPGQAPRALIY STSNKHSWTPARFSGSLLGGKAALTLSGVQPEDEAEYYCLLYYGGAQ (SEQ ID NO: 66) |
| IGLV7-46 | QAVVTQEPSLTVSPGGTVTLTCGSSTGAVTSGHYPYWFQQKPGQAPRTLIY DTSNKHSWTPARFSGSLLGGKAALTLSGAQPEDEAEYYCLLSYSGAR (SEQ ID NO: 67) |
| IGLV8-61 | QTVVTQEPSFSVSPGGTVTLTCGLSSGSVSTSYYPSWYQQTPGQAPRTLIYS TNTRSSGVPDRFSGSILGNKAALTITGAQADDESDYYCVLYMGSGI (SEQ ID NO: 68) |
| IGLV9-49 | QPVLTQPPSASASLGASVTLTCTLSSGYSNYKVDWYQQRPGKGPRFVMRV GTGGIVGSKGDGIPDRFSVLGSGLNRYLTIKNIQEEDESDYHCGADHGSGS NFV (SEQ ID NO: 69) |
| IGLV10-54 | QAGLTQPPSVSKGLRQTATLTCTGNSNNVGNQGAAWLQQHQGHPPKLLSY RNNNRPSGISERLSASRSGNTASLTITGLQPEDEADYYCSAWDSSLSA (SEQ ID NO: 70) |

This basic amino acid sequence contains a V region, and a J and a CL region are not contained. Thus, the light chain J region was assigned to the light chain variable region, with reference to the human J gene sequences (Tables 3 and 4), and the light chain constant region was assigned to the light chain variable region, with reference to the CL gene sequences (SEQ ID NOs: 84 and 85).

**[Table 3]**

| (Table 3) Kappa chain J region gene sequences | |
|---|---|
| JK1 | GTGGACGTTCGGCCAAGGGACCAAGGTGGAAATCAAAC (SEQ ID NO: 71) |
| JK2 | TGTACACTTTTGGCCAGGGGACCAAGCTGGAGATCAAAC (SEQ ID NO: 72) |
| JK3 | ATTCACTITCGGCCCTGGGACCAAAGTGGATATCAAAC (SEQ ID NO: 73) |
| JK4 | GCTCACTTTCGGCGGAGGGACCAAGGTGGAGATCAAAC (SEQ ID NO: 74) |
| JK5 | GATCACCTTCGGCCAAGGGACACGACTGGAGATTAAAC (SEQ ID NO: 75) |

**[Table 4]**

| (Table 4) Lambda chain J region gene sequences | |
|---|---|
| JL1 | TTATGTCTTCGGAACTGGGACCAAGGTCACCGTCCTAG (SEQ ID NO: 76) |
| JL2/JL3a | TGTGGTATTCGGCGGAGGGACCAAGCTGACCGTCCTAG (SEQ ID NO: 77) |
| JL3b | TTGGGTGTTCGGCGGAGGGACCAAGCTGACCGTCCTAG (SEQ ID NO: 78) |
| JL4 | TITTGTATITGGTGGAGGAACCCAGCTGATCATITTAG (SEQ ID NO: 79) |
| JL5 | CTGGGTGTTTGGTGAGGGGACCGAGCTGACCGTCCTAG (SEQ ID NO: 80) |
| JL6a | TAATGTGTCGGCATGGACCAAGTGACCGTCCTCG (SEQ ID NO: 81) |
| JL6b | TAATGTGTTCGGCAGTGGCACCAAGGTGACCGTCCTCG (SEQ ID NO: 82) |
| JL7 | TGCTGTGTTCGGAGGAGGCACCCAGCTGACCGTCCTCG (SEQ ID NO: 83) |

SEQ ID NO: 84 
TVAAPSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
SEQ ID NO: 85

QPKANPTVTLFPPSSEELQANKATLVCLISDFYPGAVTVAWKADGSPVKAGVETTKPSKQS
NNKYAASSYLSLTPEQWKSHRSYSCQVTHEGSTVEKTVAPTECS

In order to impart diversity to the light chain basic sequences mentioned above, the proportion of family gene subgroups was adjusted to match the proportion present in the living human body, and mutations or additional sequences were artificially inserted into the frame regions FR1, FR2, FR3, and FR4 and the complementarity-determining regions CDR1, CDR2, and CDR3. Specifically, with regard to the proportions of the kappa and lambda chain gene families present in the living body, the proportions of individual subgroups present in the living body were determined by referring to previous studies, etc. (Griffiths AD, et al. (1994), Prabakaran P, et al. (2012), etc.). That is to say, among the kappa chains (IGKV1 to IGKV6), the proportions of individual subgroups in the library were approximately set as follows: IGKV1: 36%, IGKV2: 11%, IGKV3: 35%, IGKV4: 11%, IGKV5: 4%, and IGKV6: 3%. Among the lambda chains (IGLV1 to IGLV10), the proportions of individual subgroups in the library were approximately set as follows: IGLV1: 40%, IGLV2: 16%, IGLV3: 21%, IGLV4: 3%, IGLV5: 6%, IGLV6: 6%, IGLV7: 2%, IGLV8: 2%, IGLV9: 2%, and IGLV10: 2%. Regarding CDR3, which is thought to be most involved in antigen recognition, the length was set as 7 to 11 amino acids wide for the kappa chain, with an average of 9.2 amino acids, and 8 to 12 amino acids wide for the lambda chain, with an average of 10.4 amino acids. The GRAVY index (Grand Average of Hydropathicity), an indicator of hydrophobicity, was set as an average of -1.00 for the kappa chain, and as an average of -0.38 for the lambda chain. Furthermore, the number of non-polar amino acids (A, C, F, G, I, L, M, P, V, W, and Y) was designed to be an average of 3.9 for the kappa chain and an average of 4.7 for the lambda chain.

### < References >

Lefranc, M.-P.; Lefranc, G. Immunoglobulins or Antibodies: IMGT™ Bridging Genes, Structures and Functions. Biomedicines 2020, 8, 319.

Prabakaran P, Chen W, Singarayan MG, Stewart CC, Streaker E, Feng Y, Dimitrov DS. Expressed antibody repertoires in human cord blood cells: 454 sequencing and IMGT/High V-QUEST analysis of germline gene usage, junctional diversity, and somatic mutations. Immunogenetics. 2012 May; 64(5): 337-50.

Foster SJ, Brezinschek HP, Brezinschek RI, Lipsky PE. Molecular mechanisms and selective influences that shape the k gene repertoire of IgM+ B cells. J Clin Invest 1997; 99: 1614-27.

Farner NL, Dorner T, Lipsky PE. Molecular mechanisms and selection influence the generation of the human V lambda J lambda repertoire. J Immunol 1999; 162: 2137-45

Griffiths AD, et al. EMBO J. 1994; 13(14): 3245-3260.

### (2) Gene synthesis

As mentioned above, with reference to the sequences of the light chain germline genes, gene sequences including light chain variable regions that are a V region and a J region, and a light chain constant region, were synthesized in the proportions set in (1) above, with 200 types of kappa chains and 200 types of lambda chains. At that time, restriction enzymes NcoI and AscI were designed at the termini as junction sites to the vector.

### (3) Library construction

In order to construct a human antibody library, samples such as normal human bone marrow-derived mononuclear cells, normal human peripheral blood-derived mononuclear cells, and human umbilical cord blood, which contain human B cells, were prepared.

### RNA extraction

To the prepared 5 to 10 x 10⁷ cells, 1 mL of TRIzol Reagent (Thermo Fisher Scientific) was added, and the resulting cells were then disintegrated. Thereafter, 50 µL of 4-Bromoanisol was added thereto. After incubation, the resultant was centrifuged, and 600 µL of an upper aqueous layer containing RNA was recovered. 70% ethanol was added, and the sample was then transferred to a spin cartridge. This spin column was washed with 350 µL of Wash Buffer I and 500 µL of Wash Buffer II, and was then eluted by adding 100 µL of RNA-free water.

### cDNA synthesis

5 µL of RNA was precipitated in ethanol, and the RNA precipitate was then suspended in a pre-mixed solution (1 µL of 50 µM Origo d(T)20 primer, 1 µL of 10 mM dNTP Mix, and 11 µL of DEPC-water). Thereafter, a pre-prepared solution (4 µL of 5XSSIV RT Buffer, 1 µL of 100 mM DTT, 1 µL of RNaseOUT Recombinant RNase Inhibitor, and 1 µL of SuperScript IV Reverse Transcriptase (200 U/µL), Thermo Fisher Scientific) was added, and the obtained mixture was reacted at 50°C for 10 minutes, at 80°C for 10 minutes. After completion of the reaction, the reaction mixture was purified using a PCR Procedure Kit (QIAGEN), and 100 µL of cDNA was eluted.

### Amplification of heavy chain fragments

Using the cDNA produced above as a template, a PCR reaction was carried out. 15 ng of the template cDNA was used, and Q5 (NEW ENGLAND BioLabs) was used as a polymerase. As primers, 7 types of forward primers (VH1 to VH7) that specifically amplify the heavy chain variable region, and 4 types of reverse primers (including 6 families; JH1-2, 3, 4-5, and 6), were used each at a final concentration of 0.5 µM (Marks JD et al. J. Mol. Biol. (1991) 222, 581-597, Campbell, M et al. Mol. Immunol. (1992) 29, 193-203.)

VH1
GTCCTCGCAACTGCGGCCCAGCCGGCCATGGCCCAGGTGCAGCTGGTGCAGTCTGG
(SEQ ID NO: 86)

VH2
GTCCTCGCAACTGCGGCCCAGCCGGCCATGGCCCAGRTCACCTTGAAGGAGTCTGGTC
C (SEQ ID NO: 87)

VH3
GTCCTCGCAACTGCGGCCCAGCCGGCCATGGCCGAGGTGCAGCTGGTGGAGTCTGG
(SEQ ID NO: 88)

VH4

GTCCTCGCAACTGCGGCCCAGCCGGCCATGGCCCAGGTGCAGCTGCAGGAGTCGGG
(SEQ ID NO: 89)

VH4-2
GTCCTCGCAACTGCGGCCCAGCCGGCCATGGCCCAGGTGCAGCTACAGCAGTGGGG
(SEQ ID NO: 90)

VH6
GTCCTCGCAACTGCGGCCCAGCCGGCCATGGCCCAGGTACAGCTGCAGCAGTCAGG
(SEQ ID NO: 91)

VH7
GTCCTCGCAACTGCGGCCCAGCCGGCCATGGCCCAGGTGCAGCTGGTGCAATCTGGG
TCTGAGT (SEQ ID NO: 92)

### Human heavy chain J region primers

| | |
|---|---|
| JH1-2 | GGTGGAGGCACTCGAGACGGTGACCAGGGTGC (SEQ ID NO: 93) |
| JH3 | GGTGGAGGCACTCGAGACGGTGACCATTGTCC (SEQ ID NO: 94) |
| JH4-5 | GGTGGAGGCACTCGAGACGGTGACCAGGGTTC (SEQ ID NO: 95) |
| JH6 | GGTGGAGGCACTCGAGACGGTGACCGTGGTCC (SEQ ID NO: 96) |

The reaction consisting of 94°C for 30 seconds, 65°C for 30 seconds, and 74°C for 2 minutes was carried out for 30 cycles, and the amplified DNA fragments were recovered. Thus, the series of operations, from RNA extraction to cDNA synthesis and heavy chain fragment amplification, was repeated to prepare antibody fragments from the prepared approximately 5 to 10 x 10⁷ immune cells.

### Construction of heavy chain library

An antibody library was constructed by incorporating the amplified DNA fragments into a vector for phage display (phagemid vector). The phagemid was designed to encode a signal sequence, a heavy chain antibody sequence, a linker sequence, a light chain antibody sequence, and an M13 phage cp3 protein, and was composed of a replication origin for E. *coli* amplification and an ampicillin resistance gene for drug selection. In order to clone the heavy chain variable region fragment into the vector, the PCR-amplified fragment was treated with the restriction enzymes Sfi-I and XhoI, while the vector was also treated similarly. Subsequently, the antibody fragment and the vector were ligated to each other, and the ligation product was then introduced into E. *coli* as follows to obtain a transformed strain. That is, the ligation product was precipitated in ethanol and was dissolved in QW. 2 µL of the obtained solution was suspended in 20 µL of DH12S (Thermo Fisher Scientific), electroporation was then performed according to the program for bacteria (25 µF, 200 Ω, 1.8 kV, and cuvette width of 0.1 cm) of the Gene Pulser Xcell (Bio-Rad). Thereafter, the entire amount of ligation product was transformed, and the genetically modified E. *coli* were cultured in a 2x YT medium containing ampicillin. After overnight culture, the cell mass was harvested, and the plasmid was purified.

### Insertion of light chain antibody gene

An antibody library was prepared by combining artificially synthesized light chain antibodies with the heavy chain antibody library prepared above. First, 400 types of synthesized light chain antibody fragments were treated with the restriction enzymes NcoI and AscI, and were then purified using a PCR Purification Kit (QIAGEN). Similarly, the heavy chain antibody library was also treated with the restriction enzymes NcoI and AscI, and vector-sized DNA fragments were cut out by agarose electrophoresis. These fragments were recovered using a GEL Extraction Kit (QIAGEN), were purified by ethanol precipitation, and were then dissolved in QW. Subsequently, the light chain fragments and the heavy chain antibody library were ligated to each other under the following conditions. That is, 150 µg of the light chain fragments were reacted with 100 µg of the heavy chain library overnight at 15°C using 10 µL of T4 Ligase (Takara). After that, they were purified by ethanol precipitation, and DNA was dissolved in QW.

2 µL of the ligation product was suspended in 20 µL of DH12S (Thermo Fisher Scientific), and electroporation was then performed according to the program for bacteria (25 µF, 200 Ω, 1.8 kV, and cuvette width of 0.1 cm) of Gene Pulser Xcell (Bio-Rad). Thereafter, the entire amount of ligation product was transformed, and the genetically modified *E*. *coli* were cultured in 2x YT medium containing ampicillin. The number of transformants was calculated by diluting the *E*. *coli* after the electroporation and plating it on agar plates, and 1 x 10¹⁰ transformants were obtained by the above-described method.

### (4) Preparation of phage antibody library

The transformed *E*. *coli* were diluted in 2 L of 2x YT medium (2x YTGA) supplemented with 0.05% glucose and 100 µg/mL ampicillin, and were cultured overnight at 30°C. After that, 625 mL of the overnight-cultured *E*. *coli* was added to 5 L of 2x YTGA, and was then cultured at 37°C. While measuring the absorbance at a wavelength of 600 nm, the cells were allowed to grow until the absorbance reached 1.0. An M13KO7 helper phage solution (NEW ENGLAND BioLabs) was added to the culture solution at an MOI of 10 per flask for infection, and the resulting cells were then cultured at 37°C for 2 hours. Thereafter, 2x YTGA was added to the culture solution to a total volume of 12 L, and the obtained mixture was cultured again overnight at 30°C. The next day, the culture solution was centrifuged at 8000 rpm for 10 minutes, and into the recovered culture supernatant, 20% polyethylene glycol/2.5 M NaCl was added was added in an amount of 1/6, followed by stirring. Thereafter, the obtained mixture was centrifuged at 8000 rpm for 20 minutes, and the precipitate was suspended in PBS to obtain a phage solution.

### < Production of antibodies against CD3 >

### (1) Production of CD3 antigen

A gene encoding 126 amino acids (amino acid residues 1-126) and a gene encoding the subsequent 8 x His tag were designed from the amino acid information (GenBank accession number NP_000724) of human CD3ε, and were artificially synthesized. This synthesized gene was inserted into a pCXN3 vector utilizing restriction enzymes to create a human CD3ε expression vector. The expression vector was transiently transfected into FreeStyle-293F cells (Invitrogen), and was then cultured with shaking for 1 week. The culture supernatant recovered from this culture was used as a sample, and was purified by passing through a His-Trap HP column (Cytiva), so as to produce an extracellular domain CD3ε-His protein.

### (2) Screening of antibodies against extracellular domain CD3ε

A screening was performed from a phage antibody library using a purified extracellular domain protein of human CD3ε. An antigen was adjusted to a concentration of 20 µg/mL with PBS, was then added in an amount of 100 µL/well to Immuno Module/Strip (NUNC), and was then left at rest overnight at 4°C. Thereafter, the antigen solution was discarded, a blocking solution (2.5% skim milk/PBS) was then added in an amount of 200 µL/well, and blocking was then performed at room temperature for 2 hours.

Subsequently, 1 x 10¹³ phage antibodies that had been adjusted with 2.5% skim milk/PBS were added to the Immuno Module/Strip from which the blocking solution had been removed, and the obtained mixture was reacted at room temperature for 2 hours. After completion of the reaction, the reaction mixture was washed 10 times with PBS, and 0.2 M Glycine HCl (pH 3.0) was added in an amount of to 100 µL/well, so as to recover phages. After that, 20 mL of *E*. *coli* DH12S with an OD of 0.5 were infected with the recovered phages for 1 hour, and thereafter, a portion thereof was seeded on an ampicillin LBG plate, and the titer of the recovered phages was then calculated. The phage-infected *E*. *coli* were cultured overnight at 30°C in 100 mL of 2x YTGA medium (2x YT, 200 µg/mL ampicillin sulfate, and 1% glucose). Thereafter, 10 mL of this overnight culture was mixed with 100 mL of 2x YTA medium (2x YT, 200 µg/mL ampicillin sulfate), and the obtained mixture was then cultured at 37°C for 1.5 hours. After that, 1 x 10¹¹ helper phage KO7 was added to the culture, and the thus obtained mixture was cultured at 37°C for 1 hour. Thereafter, 400 mL of 2x YTGAK (2x YT, 200 µg/mL ampicillin sulfate, 0.05% glucose, and 50 µg/mL kanamycin) was added, and the obtained mixture was then cultured overnight at 30°C. The obtained culture was centrifuged at 8000 rpm for 10 minutes to prepare 500 mL of supernatant, and 100 mL of PEG solution (20% polyethylene glycol 6000 and 2.5 M NaCl) was added to the supernatant, followed by fully mixing. The obtained mixture was centrifuged at 8000 rpm for 10 minutes to precipitate the phages. This precipitate was suspended in 10 mL of PBS, and a portion thereof was used to examine the number of infected *E*. *coli.* This was used as phages for the first screening.

Next, a second screening was performed using a purified extracellular domain protein of human CD3ε again. As with the first screening, the antigen was immobilized on an Immuno Module/Strip, and was then left at rest overnight at 4°C, and thereafter, the antigen solution was discarded, and blocking was performed using 200 µL/well blocking solution (2.5% skim milk/PBS) at room temperature for 2 hours. Subsequently, 1 x 10¹¹ of the 1st screening phage antibody solution that had been adjusted with 2.5% skim milk/PBS was added to the Immuno Module/Strip from which the blocking solution had been removed, and the obtained mixture was reacted at room temperature for 2 hours. After completion of the reaction, the reaction mixture was washed 20 times with PBS, and 0.2 M Glycine HCl (pH 3.0) was added in an amount of to 100 µL/well, so as to recover phages. After that, 10 mL of *E*. *coli* DH12S with an OD of 0.5 were infected with the recovered phages for 1 hour, and thereafter, a portion thereof was seeded on an ampicillin LBG plate, and the titer of the recovered phages was then calculated. The phage-infected *E*. *coli* were cultured overnight at 30°C in 50 mL of 2x YTGA medium (2x YT, 100 µg/mL ampicillin sulfate, and 1% glucose). Thereafter, 5 mL of this overnight culture was mixed with 50 mL of 2x YTA medium (2x YT, 200 µg/mL ampicillin sulfate), and the obtained mixture was then cultured at 37°C for 1.5 hours. After that, 1 x 10⁹ helper phage KO7 was added to the culture, and the thus obtained mixture was cultured at 37°C for 1 hour. Thereafter, 200 mL of 2x YTGAK (2x YT, 100 µg/mL ampicillin sulfate, 0.05% glucose, and 50 µg/mL kanamycin) was added, and the obtained mixture was then cultured overnight at 30°C. The obtained culture was centrifuged at 8000 rpm for 10 minutes to prepare 250 mL of supernatant, and 50 mL of PEG solution (20% polyethylene glycol 6000 and 2.5 M NaCl) was added to the supernatant, followed by fully mixing. The obtained mixture was centrifuged at 8000 rpm for 10 minutes to precipitate the phages. This precipitate was suspended in 50 mL of PBS, and a portion thereof was used to examine the number of infected *E. coli*. This was used as phages for the second screening.

Next, in a third screening, the antigen was immobilized in the same manner as that in the 2nd screening, and the series of procedures were repeated using 1 x 10¹⁰ of the 2nd screening phage solution.

### (3) Identification of positive clones

Positive clones were identified by ELISA using a human CD3ε extracellular domain protein. Specifically, the antigen was adjusted to 10 µg/mL with PBS, was then added in an amount of 100 µL/well to Immuno Module/Strip (NUNC), and was then left at rest at 37°C for 2 hours. After that, the antigen solution was discarded, a blocking solution (2.5% skim milk/PBS) was added in an amount of 200 µL/well, and blocking was then performed at 37°C for 2 hours. Thereafter, the blocking solution was removed, the resultant was then washed with PBS, and 100 µL/well of the monoclonal culture supernatant prepared from the above-described 3rd screening was added, followed by a reaction at 37°C for 1 hour. After washing the reaction mixture with PBS five times, 1 µg/mL Mouse anti-cp3 antibody diluted with PBS/0.05% Tween 20 was added in an amount of 100 µL/well, and the obtained mixture was reacted at 37°C for 1 hour. After washing the reaction mixture with PBS five times, 100 µL/well of HRP-labeled anti-Mouse IgG (H+L) antibody (MBL), diluted 2000-fold with PBS/0.05% Tween20, was added, and the obtained mixture was reacted at 37°C for 1 hour. After washing the reaction mixture with PBS five times, 100 µL/well of OPD in 0.1 M citrate phosphate buffer (pH 5.1) + 0.01% H₂O₂ was added, and the obtained mixture was reacted at room temperature for 5 minutes. Thereafter, 100 µL/well of 2NH₂SO₄ was added to stop the color reaction. Subsequently, the absorbance at 492 nm was measured using an Infinite F200 (TECAN). Based on these results, antibody clones that showed a positive reaction to the human CD3 antigen protein were selected, and their DNA sequences were then analyzed to identify the amino acid sequences of 6 types of antibody clones.

### < Production of antibodies against CD73 >

### (1) Production of CD73 antigen

A gene encoding 547 amino acids (amino acid residues 1-547) and a subsequent gene encoding an 8 x His tag were designed from the amino acid information (GenBank Ascension Number AAH65937) of human CD73, and were artificially synthesized. This synthesized gene was inserted into a pCXN3 vector utilizing restriction enzymes to create a human CD73 expression vector. The expression vector was transiently transfected into FreeStyle-293F cells (Invitrogen), and was then cultured with shaking for 1 week. The culture supernatant recovered from this culture was used as a sample, and was purified by passing through a His-Trap HP column (Cytiva), so as to produce a CD73-His protein.

### (2) Screening of antibodies against CD73

A screening was performed from a phage antibody library using a human CD73 antigen protein. The procedures were the same as those for the screening for CD3, and the procedures were repeated up to the 3rd screening.

### (3) Identification of positive clones

Identification of positive clones was carried out in the same manner as that of ELISA involving a reaction with the CD3 antigen. The CD73 antigen was adjusted to 10 µg/mL with PBS, and was then immobilized in an amount of 100 µL/well. Thereafter, ELISA reactivity was confirmed in a monoclonal culture supernatant prepared from the 3rd stage of CD73 screening. As a result, the sequences of positive clones were analyzed, and multiple amino acid sequences of antibody clones were identified.

### < Production of antibodies against AXL >

### (1) Production of AXL antigen

A gene encoding 451 amino acids (amino acid residues 1-451) of extracellular domain and a subsequent gene encoding an 8 x His tag were designed from the amino acid information (GenBank Ascension Number AAH32229) of human AXL, and were artificially synthesized. This synthesized gene was inserted into a pCXN3 vector utilizing restriction enzymes to create a human AXL expression vector. Expression and purification were carried out in the same manner as that for the CD3 antigen and the CD73 antigen, so as to produce an AXL-His protein.

### (2) Screening of antibodies against AXL

A screening for antibodies against the human AXL extracellular domain protein was carried out in the same manner as that for the CD3 antigen protein and the CD73 antigen protein, and multiple amino acid sequences of antibody clones reacting with human AXL were identified.

### < Identification of common light chain sequence >

### (1) Comparison of antibody sequences

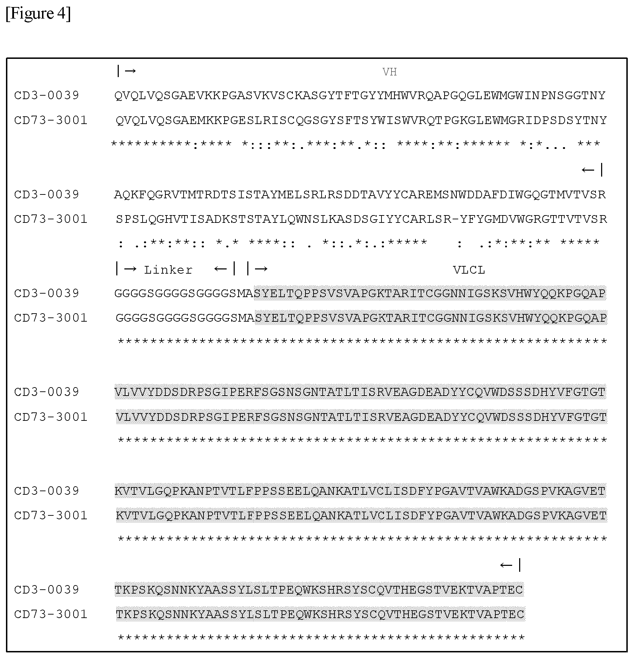

Whether mutually identical light chains existed between antibody clones reacting with the human CD3ε extracellular domain protein and antibody clones reacting with the human CD73 protein, was examined. As a result, one type of antibody clone having a completely identical light chain amino acid sequence was discovered (Figure 4).

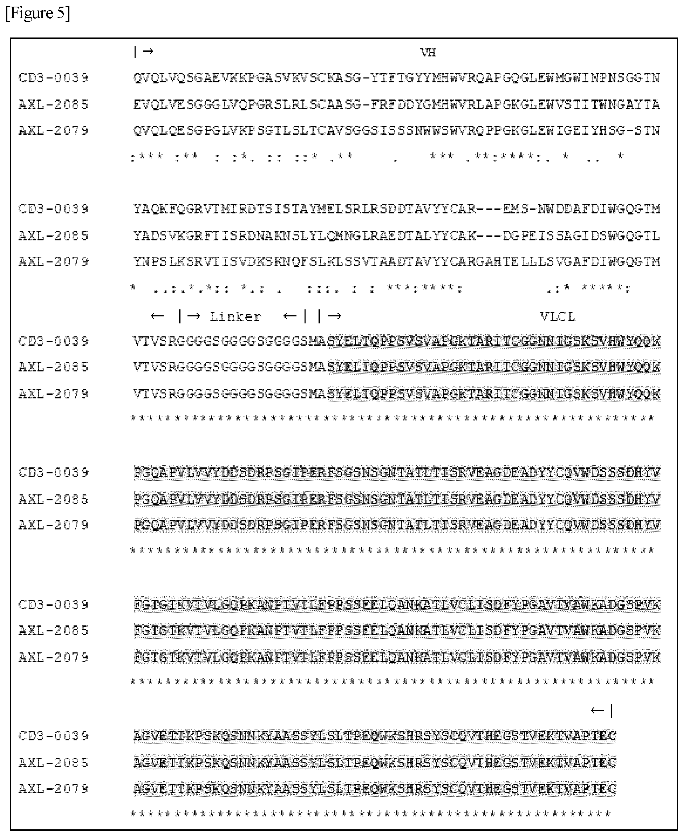

Furthermore, whether clones with a common light chain existed between antibody clones reacting with the human CD3ε extracellular domain protein and antibody clones reacting with the human AXL protein, was examined. As a result, it was found that two clones matched the anti-CD3ε antibody (Figure 5).

SEQ ID NO: 97: CD3ε antibody _0039 heavy chain variable region 
QVQLVQSGAEVKKPGASVKVSCKASGYTFTGYYMHWVRQAPGQGLEWMGWINPNSG
GTNYAQKFQGRVTMTRDTSISTAYMELSRLRSDDTAVYYCAREMSNWDDAFDIWGQGT
MVTVSR
SEQ ID NO: 98: CD3ε antibody _0039 light chain 
SYELTQPPSVSVAPGKTARITCGGNNIGSKSVHWYQQKPGQAPVLVVYDDSDRPSGIPER
FSGSNSGNTATLTISRVEAGDEADYYCQVWDSSSDHYVFGTGTKVTVLGQPKANPTVTL
FPPSSEELQANKATLVCLISDFYPGAVTVAWKADGSPVKAGVETTKPSKQSNNKYAASS
YLSLTPEQWKSHRSYSCQVTHEGSTVEKTVAPTECS
 CD3 light chain variable region sequence added
SEQ ID NO: 99: CD3ε antibody _0039 light chain variable region 
SYELTQPPSVSVAPGKTARITCGGNNIGSKSVHWYQQKPGQAPVLVVYDDSDRPSGIPER
FSGSNSGNTATLTISRVEAGDEADYYCQVWDSSSDHYVFGTGTKVTVLG
SEQ ID NO: 100: CD73 antibody_3001 heavy chain variable region 
QVQLVQSGAEMKKPGESLRISCQGSGYSFTSYWISWVRQTPGKGLEWMGRIDPSDSYTN
YSPSLQGHVTISADKSTSTAYLQWNSLKASDSGIYYCARLSRYFYGMDVWGRGTTVTVS
R
SEQ ID NO: 101: CD73 antibody_3001 light chain 
SYELTQPPSVSVAPGKTARITCGGNNIGSKSVHWYQQKPGQAPVLVVYDDSDRPSGIPER
FSGSNSGNTATLTISRVEAGDEADYYCQVWDSSSDHYVFGTGTKVTVLGQPKANPTVTL
FPPSSEELQANKATLVCLISDFYPGAVTVAWKADGSPVKAGVETTKPSKQSNNKYAASS
YLSLTPEQWKSHRSYSCQVTHEGSTVEKTVAPTECS
SEQ ID NO: 102: AXL antibody _2079 heavy chain variable region 
QVQLQESGPGLVKPSGTLSLTCAVSGGSISSSNWWSWVRQPPGKGLEWIGEIYHSGSTNY
NPSLKSRVTISVDKSKNQFSLKLSSVTAADTAVYYCARGAHTELLLSVGAFDIWGQGTMV
TVSR
SEQ ID NO: 103: AXL antibody _2079 light chain 
SYELTQPPSVSVAPGKTARITCGGNNIGSKSVHWYQQKPGQAPVLVVYDDSDRPSGIPERF
SGSNSGNTATLTISRVEAGDEADYYCQVWDSSSDHYVFGTGTKVTVLGQPKANPTVTLFP
PSSEELQANKATLVCLISDFYPGAVTVAWKADGSPVKAGVETTKPSKQSNNKYAASSYLS
 LTPEQWKSHRSYSCQVTHEGSTVEKTVAPTECS
SEQ ID NO: 104: AXL antibody_2085 heavy chain variable region 
EVQLVESGGGLVQPGRSLRLSCAASGFRFDDYGMHWVRLAPGKGLEWVSTITWNGAYT
AYADSVKGRFTISRDNAKNSLYLQMNGLRAEDTALYYCAKDGPEISSAGIDSWGQGTLVT
VSR
SEQ ID NO: 105: AXL antibody_2085 light chain

SYELTQPPSVSVAPGKTARITCGGNNIGSKSVHWYQQKPGQAPVLVVYDDSDRPSGIPERF
SGSNSGNTATLTISRVEAGDEADYYCQVWDSSSDHYVFGTGTKVTVLGQPKANPTVTLFP
PSSEELQANKATLVCLISDFYPGAVTVAWKADGSPVKAGVETTKPSKQSNNKYAASSYLS
LTPEQWKSHRSYSCQVTHEGSTVEKTVAPTECS

### < Shuffling of exogenous heavy chains and light chain library >

### (1) Preparation of heavy chain-immobilized antibody library (Cetuximab)

The heavy chain sequence was identified from the sequence information of the EGFR antibodies Cetuximab and Panitumumab, which are marketed as antibody drugs. This was used as an exogenous heavy chain and was combined with an antibody light chain library, to search for a novel light chain. In the second aspect of the present invention, even if it is a heavy chain that is not derived from the antibody library, by incorporating it into a nucleic acid library encoding a light chain, it is possible to select an antibody that functions without impairing binding activity, provided that the L chain has an optimal combination with the inserted heavy chain. Specifically, a heavy chain variable region was identified from the antibody amino acid sequence described in the CTD (Common Technical Document) for drug approval applications, and thereafter, a nucleic acid corresponding to an amino acid was designed and artificial synthesis was performed. This heavy chain variable region sequence was inserted into a library containing a nucleic acid encoding a light chain, and a light chain library was completed in which the heavy chain was immobilized in the variable region of Cetuximab.

More specifically, 117 amino acids (SEQ ID NO: 106) were obtained from the CTD, and the artificially synthesized DNA fragment was ligated to a vector containing an L chain library by a restriction enzyme treatment. That is to say, 5 µg of the heavy chain fragment was reacted with 5 µg of the vector using 2 µL of T4 Ligase (Takara Inc.) at 15°C for 4 hours. Thereafter, the resultant was purified by ethanol precipitation, and the plasmid DNA was dissolved in QW.
SEQ ID NO: 106: Cetuximab heavy chain variable region

QVQLKQSGPGLVQPSQSLSITCTVSGFSLTNYGVHWVRQSPGKGLEWLGVIWSGGNTDY
NTPFTSRLSINKDNSKSQVFFKMNSLQSNDTAIYYCARALTYYDYEFAYWGQGTLVSS

Subsequently, gene transfer of the ligation product and preparation of the phage antibody library were carried out according to the contents described in the above examples, and a phage solution was prepared.

### (2) Antibody screening (Cetuximab)

In order to select clones that react with the EGFR protein from a light chain library immobilized on the heavy chain of the prepared Cetuximab, screening procedures were performed. Specifically, a recombinant protein (GenScript) was adjusted to a concentration of 20 µg/mL using PBS, so as to prepare an antigen at, and the antigen was then added in an amount of 100 µL/well to Immuno Module/Strip (NUNC), followed by leaving at rest overnight at 4°C. Thereafter, the antigen solution was discarded, 200 µL/well of blocking solution (2.5% skim milk/PBS) was then added, and blocking was performed at room temperature for 2 hours. Next, 1 x 10¹⁰ phage antibodies that had been adjusted with 2.5% skim milk/PBS were added to the Immuno Module/Strip, from which the blocking solution had been removed, and the mixture was reacted at room temperature for 2 hours. After completion of the reaction, the reaction mixture was washed 10 times with PBS, and 0.2 M glycine HCl (pH 3.0) was added in an amount of 100 µL/well, so as to recover phages. After that, 20 mL of *E*. *coli* DH12S with an OD of 0.5 were infected with the recovered phages for 1 hour, and a portion thereof was then seeded on an ampicillin LBG plate. The titer of the recovered phages was then calculated. The phage-infected *E*. *coli* were cultured overnight at 30°C in 100 mL of 2x YTGA medium (2x YT, 200 µg/mL ampicillin sulfate, and 1% glucose). Thereafter, 10 mL of this overnight culture was mixed with 100 mL of 2x YTA medium (2x YT, 200 µg/mL ampicillin sulfate), and the obtained mixture was then cultured at 37°C for 1.5 hours. After that, 1 x 10¹¹ helper phage KO7 was added to the culture, and the thus obtained mixture was cultured at 37°C for 1 hour. Thereafter, 400 mL of 2x YTGAK (2x YT, 200 µg/mL ampicillin sulfate, 0.05% glucose, and 50 µg/mL kanamycin) was added, and the obtained mixture was then cultured overnight at 30°C. The obtained culture was centrifuged at 8000 rpm for 10 minutes to prepare 500 mL of supernatant, and 100 mL of PEG solution (20% polyethylene glycol 6000 and 2.5 M NaCl) was added to the supernatant, followed by fully mixing. The obtained mixture was centrifuged at 8000 rpm for 10 minutes to precipitate the phages. This precipitate was suspended in 10 mL of PBS, and a portion thereof was used to examine the number of infected *E*. *coli.* This was used as phages for the first screening.

Similarly, in the 2nd screening, the antigen was immobilized in the same manner as that in the 1st screening, and the series of procedures were repeated using 1 x 10⁹ of the 1st screening phage solution, while the number of washing procedures was changed to 15 times. Furthermore, in the 3rd screening, the antigen was immobilized as before, 1 x 10⁹ of the 2nd phage solution was used, and the number of washing procedures was changed to 25 times, and the screening was performed.

### (3) Identification of positive clones (Cetuximab)

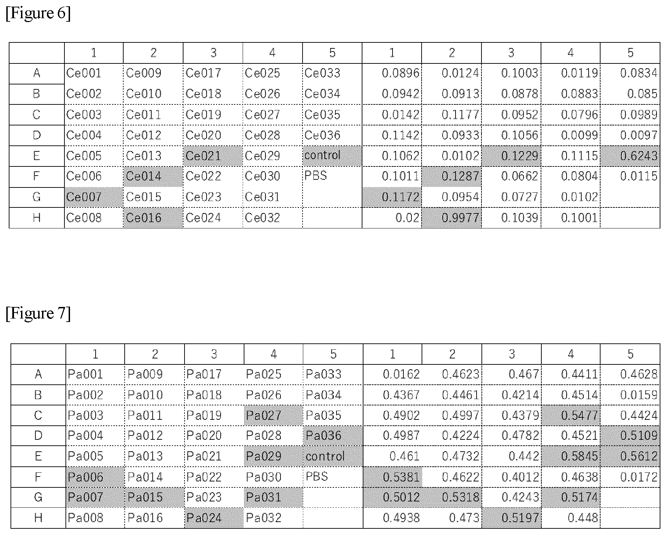

Positive clones were identified by ELISA using a human EGFR extracellular domain protein. Specifically, the antigen was adjusted to 10 µg/mL with PBS, was then added in an amount of 100 µL/well to Immuno Module/Strip (NUNC), and was then left at rest at 37°C for 2 hours. After that, the antigen solution was discarded, a blocking solution (2.5% skim milk/PBS) was added in an amount of 200 µL/well, and blocking was then performed at 37°C for 2 hours. Thereafter, the blocking solution was removed, the resultant was then washed with PBS, and 100 µL/well of the monoclonal culture supernatant prepared from the above-described 3rd screening was added, followed by a reaction at 37°C for 1 hour. After washing the reaction mixture with PBS five times, 1 µg/mL Mouse anti-cp3 antibody diluted with PBS/0.05% Tween 20 was added in an amount of 100 µL/well, and the obtained mixture was reacted at 37°C for 1 hour. After washing the reaction mixture with PBS five times, 100 µL/well of HRP-labeled anti-Mouse IgG (H+L) antibody (MBL), diluted 2000-fold with PBS/0.05% Tween20, was added, and the obtained mixture was reacted at 37°C for 1 hour. After washing the reaction mixture with PBS five times, 100 µL/well of OPD in 0.1 M citrate phosphate buffer (pH 5.1) + 0.01% H₂O₂ was added, and the obtained mixture was reacted at room temperature for 5 minutes. Thereafter, 100 µL/well of 2NH₂SO₄ was added to stop the color reaction. Subsequently, the absorbance at 492 nm was measured using an Infinite F200 (TECAN). Based on the obtained results, phage clones showing a positive reaction to the human EGFR antigen protein were selected, and the DNA sequences of the top four clones were analyzed based on the signal intensity of the ELISA reaction. The four clones were classified into the top one clone and the remaining three clones, and ultimately, the amino acid sequences of two types of antibody clones were identified. From these results, it was revealed that clones possessing a heavy chain derived from Cetuximab and combining it with a novel sequence selected from a light chain library can be obtained while maintaining reactivity to the EGFR protein (Figure 6). Moreover, it was also confirmed that one clone has a higher signal intensity compared to the scFv-type Cetuximab used as a control.

### (4) Identification of L chain sequences (Cetuximab)

The light chain sequences confirmed by ELISA are shown below.

SEQ ID NO: 107: Ce007 light chain
EIVLTQSPATLSLSPGERATLSCRASQSVSGFLAWYQQKPGQAPRLLIYDTSNRATGIPARFS
GSGSGADFTLTISSLESEDLAVYYCQQRSAWPITFGQGTRLEIKRTVAAPSVFIFPPSDEQLK
SGTASVVCLLNNFYPREAKVQWKVDNALQSGNSQESVTEQDSKDSTYSLSSTLTLSKAD
YEKHKVYACEVTHQGLSSPVTKSFNRGEC

SEQ ID NO: 108: Ce016 light chain
ETTLTQSPATLSVSPGERATLSCRASQSVSSNLAWYQQKPGQAPRLLIYGASTRATGIPARF
SGSGSGKEFTLTISSLQSEDFAVYYCQQYNNWPLTFGQGTRLEIKRTVAAPSVFIFPPSDEQL
KSGTASVVCLLNNFYPREAKVQWKVDNALQSGNSQESVTEQDSKDSTYSLSSTLTLSKA
DYEKHKLYACEVTHQGLSSPVTKSFNRGEC

### (5) Production of heavy chain-immobilized library (Panitumumab)

The sequence of Panitumumab was obtained from the CTD according to the method described in (1) above, and the sequence of a heavy chain variable region was synthesized.

SEQ ID NO: 109: Panitumumab heavy chain variable region



Moreover, according to the above-described method, a light chain library immobilized on the heavy chain of Panitumumab was produced.

### (6) Antibody screening (Panitumumab)

Screening was performed on the EGFR protein according to the method described in (2) above.

### (7) Identification of positive clones (Panitumumab)

According to the procedures described in (3) above, monoclonal antibodies were produced at the stage where the 3rd screening was completed, and positive clones were identified using ELISA (Figure 7). As a result, since many of the 36 clones tested positive, the DNA sequences of the top 8 clones were analyzed.

### (8) Identification of L chain sequences (Panitumumab)

As a result of the sequence analysis, it was revealed that the top 8 clones were classified into two types of sequences.

SEQ ID NO: 110: Pa006 light chain
DIQMTQSPSSLSASVGDRVTITCQASQDISRYLNWYQQKPGKAPELLIYDASDLETGVPSR
FSGSGSGTGFTFTISSLQPEDIATYYCQQYDTLPLTFGGGTKVEIKRTVAAPSVFIFPPSDEQL
KSGTASVVCLLNNFYPREAKVQWKVDNALQSGNSQESVTEQDSKDSTYSLSSTLTLSKA
DYEKHKVYACEVTHQGLSSPVTKSFNRGEC

SEQ ID NO: 111: Pa007 light chain

DIQMTQSPSSLSASVGDRVTITCQASQDISNYLNWYQQKPGKAPKLLIYDASNLETGVPSR
FSGSGSGTDFTFTISSLQPEDIATYYCQQYDNLPLTFGGGTKVEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC

### < Isolation of bispecific antibody >

An antibody that binds to a transferrin receptor (TfR) is created as an antibody that becomes a counterpart to the antibody that binds to the EGFR protein. Specifically, in order to pair with the combination of a heavy chain derived from Cetuximab and a light chain derived from the light chain library, a light chain found by combining the anti-TfR antibody derived from the heavy chain library with the above-described Cetuximab heavy chain is used as a common light chain, and a heavy chain having binding activity to TfR is searched.

### (1) Production of TfR protein

A gene encoding the amino acid residues 89 to 760 and the subsequent gene encoding the His tag were designed from the amino acid information (GenBank Ascension Number NP_003225.2) of human TfR, and were artificially synthesized. This synthesized gene was inserted into a pCXN3 vector utilizing restriction enzymes to create a human TfR expression vector. The expression vector was transiently transfected into FreeStyle-293F cells (Invitrogen), followed by 1 week of shaking culture. The culture supernatant recovered from this culture was used as a sample, and was then purified by passing it through a His-Trap HP column (Cytiva), so as to produce an extracellular domain TfR-His protein.

### (2) Antibody screening for TfR protein

Screening for the TfR protein was performed according to the method described above in the screening for the EGFR protein. Specifically, three rounds of screening for the TfR protein were performed using the antibody library prepared in "(4) Preparation of phage antibody library" above, and multiple clones for TfR were identified

### (3) Identification of common light chains

From the antibody clone population that reacted with the TfR protein, sequences identical to the light chain newly combined with the heavy chain of Cetuximab were selected. As a result, the light chain sequence of Ce016 and the light chain sequence of TfR672 were completely identical to each other.
SEQ ID NO: 112: TfR672 heavy chain variable region 
EVQLVESGGGLVQPGGSLRLSCSASGFSFSDYAMQWVRQAPGKGLEYVAAISHEGGTTY
YADSVKGRFIISRDNSENTVHLQMSGLRRDDTALYYCVQDRFYYGSQSETHNWGQGTLV
TVSS
SEQ ID NO: 113: TfR672 light chain

ETTLTQSPATLSVSPGERATLSCRASQSVSSNLAWYQQKPGQAPRLLIYGASTRATGIPARF
SGSGSGKEFTLTISSLQSEDFAVYYCQQYNNWPLTFGQGTRLEIKRTVAAPSVFIFPPSDEQL
KSGTASVVCLLNNFYPREAKVQWKVDNALQSGNSQESVTEQDSKDSTYSLSSTLTLSKA
DYEKHKLYACEVTHQGLSSPVTKSFNRGEC

Furthermore, from the antibody population that reacted with the TfR protein, sequences identical to the light chain newly combined with the heavy chain of Panitumumab were selected. As a result, the light chain sequence of Pa007 and the light chain sequence of TfR443 were completely identical to each other.
SEQ ID NO: 114: TfR443 heavy chain variable region 
QVQLVQSGAEVKKPGASVKVSCKASGYTFATYGMHWVRQAPGQSLEWMGWINVGSGD
TEYSQKFQGRVTITRDTSASTAYMELSSLRFEDTAVYYCARDNGRWGQGTLVTVSS
SEQ ID NO: 115: TfR443 light chain

DIQMTQSPSSLSASVGDRVTITCQASQDISNYLNWYQQKPGKAPKLLIYDASNLETGVPSR
FSGSGSGTDFTFTISSLQPEDIATYYCQQYDNLPLTFGGGTKVEIKRTVAAPSVFIFPPSDEQ
LKSGTASVVCLLNNFYPREAKVQWKVDNALQSGNSQESVTEQDSKDSTYSLSSTLTLSKA
DYEKHKVYACEVTHQGLSSPVTKSFNRGEC

### (4) Confirmation of reactivity

The ELISA activity of TfR672 and TfR443 was measured. Specifically, the TfR protein was used as an antigen, and the same method as the ELISA against the EGFR protein was carried out.

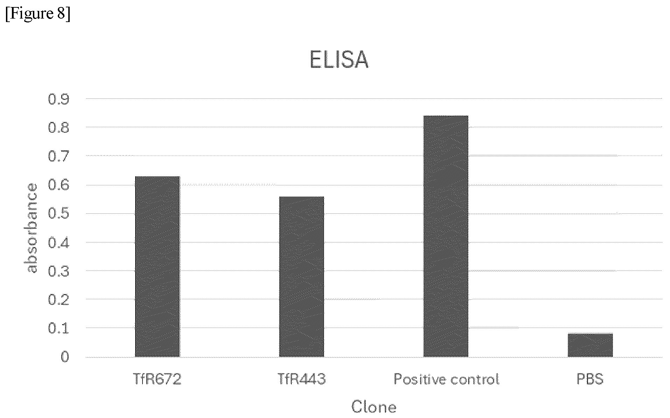

As a result, it was confirmed that TfR672 and TfR443 have binding activity to the TfR protein (Figure 8).

That is, by combining two antibody fragments, namely, one antibody fragment having a Cetuximab heavy chain and a Ce016 light chain, and the other antibody fragment consisting of a TfR672 heavy chain and a TfR672 light chain (same sequence as the Ce016 light chain), a bispecific antibody (recognizing EGFR and TfR) having a common light chain can be produced.

Similarly, by combining two antibody fragments, namely, one antibody fragment having a Panitumumab heavy chain and Pa007, and the other antibody fragment consisting of a TfR443 heavy chain and a TfR443 light chain (the same sequence as that of the Pa007 light chain), a bispecific antibody (recognizing EGFR and TfR) having a common light chain can be produced.

### (5) Consideration

Cetuximab, marketed as an antibody against the EGFR protein, was obtained by mouse immunization, and is a chimeric antibody in which the Fc portion was replaced with human Fc through genetic modification. Therefore, the variable region retains the mouse antibody gene. On the other hand, the light chain library used in this study is constructed utilizing human naive sequences. According to the examples of the present invention, it is suggested that combinations of sequences, in which reactivity is not lost even when a heavy chain variable region derived from a mouse antibody is combined with a light chain derived from a human antibody, can be discovered, and it is considered that it provides extremely great significance to the creation of common light chains for bispecific antibodies.

Furthermore, Panitumumab is an antibody produced by Amgen in the U.S.A., utilizing Xenomouse technology, and Panitumumab has been isolated from human antibody-producing mice, into which human antibody genes had been inserted. In recent years, many medicaments marketed as complete human antibodies are believed to be derived from such human antibody-producing mice. Also, from the fact that a common light chain is obtained both with respect to the Panitumumab heavy chain obtained by immunizing these human antibody-producing mice and with respect to the light chain derived from the library suggests that the library-derived light chain possesses a versatile group of sequences. The fact that multiple light chains can be isolated with respect to heavy chains other than those derived from the library suggests that the common light chain could potentially be used to create bispecific antibodies derived from two types of heavy chains other than those from the library.

## Claims

1. A method for identifying a common L chain for use in production of a multispecific antibody, comprising:
(1) a step of producing a first library containing multiple antibodies that recognize a first antigen,
(2) a step of producing a second library containing multiple antibodies that recognize a second antigen, and
(3) a step of selecting an antibody that recognizes the first antigen and an antibody that recognizes the second antigen, which have a common L chain, from the first library and the second library.

2. The method according to claim 1, wherein
the step (1) is a step of selecting multiple antibodies that recognize a first antigen from a library containing multiple antibodies having H chains and L chains, so as to produce a first library, and
the step (2) is a step of selecting multiple antibodies that recognize a second antigen from a library containing multiple antibodies having H chains and L chains, so as to produce a second library.

3. The method according to claim 2, wherein the library containing multiple antibodies having H chains and L chains contains 10⁹ types or more antibodies.

4. The method according to claim 2, wherein the type of H chains in the library containing multiple antibodies having H chains and L chains is 10⁶ or more.

5. The method according to claim 2, wherein the type of L chains in the library containing multiple antibodies having H chains and L chains is 10³ or less.

6. The method according to claim 2, wherein the ratio between the types of H chains and the types of L chains in the library containing multiple antibodies having H chains and L chains is 1000 times or more.

7. The method according to claim 1, wherein
the step (1) comprises:
(1-A) a step of incorporating nucleic acids encoding the H chains of the antibodies that recognize a first antigen into a library of nucleic acids encoding the L chains, to produce an antibody library containing the H chains and L chains of the antibodies that recognize the first antigen, and
(1-B) a step of selecting the antibodies that recognize the first antigen from the antibody library obtained in the step (1-A), and
the step (2) comprises:
(2-A) a step of incorporating nucleic acids encoding the H chains of the antibodies that recognize a second antigen into a library of nucleic acids encoding the L chains, to produce an antibody library containing the H chains and L chains of the antibodies that recognize the second antigen, and
(2-B) a step of selecting the antibodies that recognize the second antigen from the antibody library obtained in the step (2-A).

8. The method according to claim 7, wherein the types of L chains in the antibody library obtained in the step (1-A) and in the antibody library obtained in the step (2-A) are each 10³ or less.

9. The method according to claim 1, wherein
the step (1) is a step of selecting multiple antibodies that recognize a first antigen from a library containing multiple antibodies having H chains and L chains, so as to produce a first library, and
the step (2) comprises:
(2-A) a step of incorporating nucleic acids encoding the H chains of the antibodies that recognize a second antigen into a library of nucleic acids encoding the L chains, to produce an antibody library containing the H chains and L chains of the antibodies that recognize the second antigen, and
(2-B) a step of selecting the antibodies that recognize the second antigen from the antibody library obtained in the step (2-A).

10. The method according to claim 1, wherein the first antigen and the second antigen are each selected from a cancer antigen, an immune cell antigen, a bioactive molecule, and an enzyme.

11. A method for producing a multispecific antibody, comprising:
a step of identifying an L chain by the method according to any one of claims 1 to 10, and
a step of producing a multispecific antibody, using the L chain identified above, the H chain recognizing the first antigen, and the H chain recognizing the second antigen.
